# FASCICULE DE BREVET EUROPEEN

(11) **EP 3 700 431 B1**
(45) Date de publication et mention de la délivrance du brevet: **08.12.2021**
(21) Numéro de dépôt: 18788776.5
(22) Date de dépôt: 24.10.2018
(51) Int. Cl.: A61B 10/00

(54) **DISPOSITIF DE PRÉLÈVEMENT DE FLUIDE INTESTINAL**
VORRICHTUNG ZUM SAMMELN VON DARMFLÜSSIGKEIT
DEVICE FOR COLLECTING INTESTINAL FLUID

(30) Priorité: 25.10.2017 FR 1760069
(43) Date de publication de la demande: 02.09.2020
(73) Titulaire: Université Grenoble Alpes, 38400 Saint-Martin-d'Hères (FR); Centre National de la Recherche Scientifique, 75016 Paris (FR); Centre Hospitalier Universitaire Grenoble Alpes, 38700 La Tronche (FR)
(72) Inventeur: THELU, Jacques, 38920 Crolles (FR); CINQUIN, Philippe, 38330 Saint Nazaire Les Eymes (FR); MARTIN, Donald Keith, 38610 Gieres (FR); SORANZO, Thomas, 38000 Grenoble (FR); ALCARAZ, Jean-Pierre, 38530 Pontcharra (FR); KWETER, Jean-Marc, 40000 Mont De Marsan (FR)
(74) Mandataire: Bronchart, Quentin
(86) Numéro de dépôt international: PCT/EP2018/079077
(87) Numéro de publication internationale: WO 2019/081539

(56) Documents cités:
- DE-A1- 19 801 573
- US-A- 3 688 763
- US-A- 5 971 942
- US-A1- 2007 173 738

## Description

La présente invention concerne un dispositif de prélèvement d'un échantillon de fluide intestinal, chez l'humain ou l'animal, notamment à des fins de recherche et de diagnostic.

La connaissance du système digestif et de son microbiote est considérée comme un sujet d'intérêt majeur en santé humaine ou animale. En effet, en termes de diagnostic de pathologies, il est indispensable de pouvoir caractériser de manière qualitative et quantitative le microbiote contenu dans différents endroits du système digestif, en particulier dans les intestins.

A l'heure actuelle, la majorité des analyses est effectuée sur les fèces, restreignant donc les possibilités d'observation du microbiote. Alors que les méthodes d'analyses actuelles sont constamment améliorées et moins onéreuses, il existe un besoin d'un moyen autonome, sans effet négatif sur la santé, simple et peu onéreux permettant de prélever le microbiote du fluide intestinal.

Des dispositifs de prélèvement de fluide intestinal sont connus, notamment des demandes DE 198 01 573 et US 5 971 942 qui décrivent des capsules de prise d'échantillon formées d'une enveloppe délimitant un volume intérieur dans lequel règne une pression intérieure basse, le liquide intestinal étant alors aspiré à l'intérieur de la capsule à travers un orifice par la différence de pression, et la fermeture de la capsule étant obtenue par l'équilibre des pressions hors et dans la capsule.

Le dispositif de US 5 971 942 est caractérisé par un volume interne faible et une pression interne faibles. Le dispositif de DE 198 01 573 comprend un mécanisme de fermeture d'un orifice comprenant une mousse qui n'est pas à l'état compressé.

Ainsi, la présente invention a pour objectif de fournir un dispositif de prélèvement d'un échantillon de fluide intestinal à différents niveaux dans les intestins.

Un autre objectif de la présente invention est de fournir un tel dispositif qui permette de recueillir des volumes variables, tout en restant facile à avaler par le patient.

Un autre objectif de la présente invention est de fournir un tel dispositif qui soit peu onéreux et facilement utilisable et récupérable.

D'autres objectifs de la présente invention apparaîtront à la lecture de la description qui suit.

A cet effet, la présente invention concerne un dispositif de prélèvement d'un échantillon de fluide intestinal comprenant :
- un matériau interne en polymère :
   - présentant une structure alvéolaire ouverte au moins partiellement compressée, et
   - ayant un volume Vᵢ,
- une enveloppe en polymère élastique et étanche au fluide digestif, ladite enveloppe comprenant au moins un orifice,
- un matériau gastro-résistant et susceptible de se dissoudre dans le fluide intestinal à 37°C étant disposé au-dessus de chaque orifice; et
- au moins un mécanisme de fermeture de l'(des) orifice(s).

L'admission du fluide intestinal au sein du dispositif provoque le gonflement du matériau interne qui induit la fermeture du mécanisme de fermeture de l'(les)orifice(s).

Le matériau interne est susceptible de gonfler à un volume supérieur ou égal à 1,1 × Vi par absorption de fluide intestinal.

L'enveloppe enveloppe le matériau interne, ledit au moins un orifice permettant l'admission du fluide intestinal au sein du matériau interne.

Ainsi, le gonflement du matériau interne provoque une déformation élastique de l'enveloppe.

L'invention permet ainsi la (pré)détermination de manière précise du volume de liquide intestinal (à prélever) prélevé, du fait que la force de gonflement du matériau interne s'équilibre avec la force exercée par l'enveloppe du fait de sa déformation élastique.

Par « fluide intestinal », on entend le fluide présent dans les intestins, ce fluide comprenant un très grand nombre de germes vivants, autrement appelés le « microbiote ». Par « fluide digestif », on entend le fluide gastrique ou intestinal.

Ci-après, l'expression « compris entre X et Y » signifie que les bornes X et Y sont inclues. Par « en polymère », on entend comprenant un polymère.

De préférence, le matériau interne en polymère compris dans le dispositif présente une solubilité dans le fluide intestinal mesurée à 37°C inférieure ou égale à 1% massique. De préférence le matériau interne est insoluble dans le fluide intestinal à 37°C. Par « insoluble » on entend que le matériau interne ne se dissout pas (solubilité de 0% massique) après immersion dans le fluide intestinal à 37°C pendant la durée du transit intestinal, de préférence pendant au moins 24 heures.

De manière avantageuse, le polymère du matériau interne est choisi parmi les poly (alcool vinylique) au moins partiellement réticulés, les acides poly(méth)acryliques au moins partiellement réticulés et leurs sels, les polysaccharides au moins partiellement réticulés, ou un mélange de ceux-ci. Parmi les polysaccharides, on peut citer le dextrane ou l'un de ses dérivés.

La réticulation au moins partielle de ces polymères assure que leur solubilité à 37°C dans le fluide intestinal soit inférieure ou égale à 1% massique, de préférence 0% (insolubles). En effet, les polymères mentionnés ci-dessus non réticulés sont généralement solubles dans l'eau, et donc dans le fluide intestinal à 37°C.

Le matériau interne en polymère a une structure alvéolaire ouverte et un volume Vᵢ qui correspond à son volume avant ingestion. Il se présente donc sous la forme d'une mousse ou d'une éponge.

La structure alvéolaire ouverte du matériau interne comprend des alvéoles séparées les unes des autres par des parois en polymère, l'épaisseur moyenne desdites parois étant de préférence comprise entre 2 µm et 500 µm. L'épaisseur moyenne des parois des alvéoles est mesurée par microscopie électronique à balayage.

Les alvéoles de la structure alvéolaire ouverte sont généralement au moins partiellement, voire totalement, remplies de gaz, de préférence d'air. Elles peuvent comprendre de l'eau.

La structure alvéolaire ouverte est au moins partiellement compressée, ce par quoi le matériau interne est susceptible de gonfler par absorption de fluide intestinal, à un volume supérieur ou égal à 1,1 x Vᵢ, notamment supérieur ou égal à 2 x Vᵢ, de préférence supérieur ou égal à 6 x Vᵢ.

Par « enveloppe élastique », on entend que l'enveloppe a une élasticité telle qu'elle permet le gonflement du matériau interne à un volume supérieur ou égal à 1,1 x Vᵢ, de préférence à un volume de 2 x Vᵢ à 6 x Vᵢ. Typiquement, l'enveloppe en polymère est étirable à 25°C sous une force de 1 N sur une longueur comprise entre 2 fois et 6 fois sa longueur initiale. De préférence, l'enveloppe élastique présente une dureté comprise entre 30 et 55 Shore (méthode normée shore A). De préférence, le polymère de l'enveloppe a un module de Young compris entre 1,1 et 1,9 MPa.

De préférence, le polymère de l'enveloppe est choisi parmi le latex, les polyvinyles, les polymères nitrile, le silicone, et un mélange de ceux-ci, de préférence le polymère de l'enveloppe est choisi parmi les polymères nitrile tel qu'un copolymère butadiène-acrylonitrile.

Puisque l'enveloppe enveloppe le matériau interne, le gonflement du matériau interne une fois qu'il est ingéré est limité par l'élasticité de l'enveloppe. De préférence, avant ingestion, l'enveloppe est à l'état non étiré.

Par « enveloppe étanche au fluide digestif », on entend qu'à 37°C, excepté au niveau de l'(les) orifice(s), le fluide digestif ne passe pas à travers l'enveloppe. L'enveloppe est donc imperméable au fluide digestif. Généralement, l'enveloppe présente des pores de diamètre moyen tel que mesuré par microscopie électronique à balayage inférieur à 0,2 µm. De préférence, l'enveloppe est exempte de pores.

De préférence, l'enveloppe est stable vis-à-vis du fluide digestif. Par « stable » on entend que l'enveloppe ne se désintègre pas, sa composition chimique et son étanchéité ne se modifient pas au contact du fluide digestif pendant la durée du transit intestinal, de préférence pendant au moins 24 heures.

L'enveloppe définit un espace interne, dans lequel le matériau interne est disposé. Le matériau interne occupe généralement au moins 80%, notamment au moins 90%, de préférence au moins 95% du volume de l'espace interne formé par l'enveloppe. De préférence la différence entre la pression au sein de l'enveloppe (et donc au sein du matériau interne qu'elle enveloppe) et la pression atmosphérique est inférieure à 5%, notamment inférieure à 1%, de préférence 0%. Généralement, la pression au sein de l'enveloppe (et donc au sein du matériau interne qu'elle enveloppe) est la pression atmosphérique (1 bar).

De préférence, l'enveloppe comprend 1, 2, 3, 4 ou 5 orifices, de préférence un seul orifice. L'orifice permet l'admission du fluide intestinal au sein du dispositif, et donc au sein du matériau interne.

L'enveloppe peut comprendre un fil métallique permettant de renforcer l'enveloppe pour qu'elle résiste mieux aux mouvements péristaltiques. Ce fil métallique peut être disposé dans l'épaisseur de l'enveloppe. De préférence, c'est un fil métallique ferromagnétique.

Des marqueurs colorés peuvent être intégrés à la surface de l'enveloppe pour permettre la reconnaissance des dispositifs récupérés. Une puce en silicium, une puce RFID ou analogue peuvent également être utilisées.

Par « matériau gastro-résistant » on entend un matériau qui ne se délite pas et ne se dissout pas au contact du fluide gastrique. Par « susceptible de se dissoudre dans le fluide intestinal à 37°C », on entend que la limite de solubilité du matériau gastro-résistant est supérieure à 95% massique, de préférence supérieure à 98% massique dans le fluide intestinal à 37°C.

De préférence, le matériau gastro-résistant est susceptible de se dissoudre dans le fluide intestinal à un pH donné. En effet, la solubilité du matériau gastro-résistant peut essentiellement dépendre du pH du milieu dans lequel il se trouve. Ainsi, de préférence, le matériau gastro-résistant est insoluble à 37°C en présence d'un pH acide (inférieur à 7), de préférence il est insoluble à 37°C à un pH inférieur à 5. Par « insoluble », on entend que la limite de solubilité du matériau gastro-résistant à un pH inférieur à 5 est inférieure ou égale à 1% massique, de préférence égale à 0% massique. Le pH du fluide intestinal varie de 5 à 8 en fonction de l'endroit exact de l'intestin où il se situe. Il est connu de l'homme du métier que l'estomac à jeun est caractérisé par un pH compris entre 1,5 et 3, ce pH étant compris entre 2 et 5 après un repas et progressivement neutralisé au sortir de l'estomac. Ainsi, le pH au niveau du duodénum est compris entre 5,4 et 6,1, le pH au niveau de l'iléon est compris entre 7 et 8, le caecum et le colon présentent un pH compris entre 5,5 et 7, et enfin le rectum présente un pH égal à 7. Ainsi, l'homme du métier comprendra aisément que le matériau gastro-résistant est choisi en fonction de sa solubilité au pH à l'endroit où le prélèvement de fluide intestinal sera réalisé. De préférence, un matériau gastro-résistant et susceptible de se dissoudre dans le fluide intestinal à 37°C est disposé au-dessus de la totalité du dispositif. Par exemple, une gélule gastro résistante susceptible de se dissoudre dans le fluide intestinal à 37°C peut recouvrir l'ensemble formé par l'enveloppe et le matériau interne. Le matériau gastro-résistant est susceptible de se dissoudre totalement dans le fluide intestinal à 37°C lorsque le pH atteint une valeur déterminée. De préférence, le matériau gastro-résistant se dissout rapidement, en une durée inférieure à 10min, de préférence inférieure à 5min.

De préférence, le matériau gastro-résistant est choisi parmi le poly(méthacrylate de méthyle), l'acétophtalate de cellulose, la carboxyméthylcellulose, l'acéto-succinate d'hypromellose, l'acétate phtalate de polyvinyle, les gommes laques, etc. De manière avantageuse, le matériau gastro-résistant est choisi parmi les composés Eudragit^{®} L30 D-55 et Eudragit^{®} L100-55 commercialisés par la société Evonik^{©}

Avantageusement, une ou plusieurs couches intermédiaire(s), typiquement une couche en alginate, peut(vent) être disposé(es) entre l'orifice et le matériau gastro-résistant. De préférence, aucune couche intermédiaire n'est présente entre l'orifice et le matériau gastro-résistant.

Dans un mode de réalisation, l'enveloppe et le matériau interne sont enveloppés dans une gélule en alginate, ladite gélule étant recouverte de matériau gastro-résistant tel que ceux susmentionnés. La gélule en alginate est complètement soluble à 37°C dans le fluide intestinal, de préférence la limite de solubilité de la gélule en alginate dans le fluide intestinal à 37°C est supérieure à 95% massique. L'extérieur de la gélule est rendu gastro-résistant, notamment grâce à un pelliculage par un matériau gastro-résistant.

De manière avantageuse, le dispositif peut être sous forme cylindrique, sphérique ou ellipsoïde, de préférence cylindrique. De préférence, le dispositif peut être un cylindre plat, sous forme de demi-lune.

De préférence, le dispositif est susceptible de prélever un volume de fluide intestinal compris entre 0,01 mL et 1 mL, de préférence un volume de fluide intestinal égal à 0,3 mL.

Le mécanisme de fermeture de l'(les) orifice(s) permet de fermer chaque orifice de l'enveloppe en l'(les)obstruant. Le dispositif peut comprendre autant de mécanismes de fermeture d'orifice qu'il y a d'orifices. Alternativement, un seul mécanisme de fermeture peut permettre de fermer plusieurs orifices.

Les différents mécanismes de fermeture ci-dessous sont décrits pour la fermeture d'un seul orifice mais peuvent être utilisés pour en fermer plusieurs. Dans le cas où l'enveloppe comprend plusieurs orifices, des mécanismes de fermeture identiques ou différents peuvent être utilisés.

Différents mécanismes de fermeture de l'(les) orifice(s) du dispositif vont maintenant être décrits plus en détails à l'aide de modes de réalisation pris à titre d'exemples non limitatifs se référant aux figures annexées dans lesquelles :
- la figure 1 représente un premier mode de réalisation du mécanisme de fermeture, ledit mécanisme comprenant un clapet ;
- la figure 2 représente une première alternative du deuxième mode de réalisation du mécanisme de fermeture, ledit mécanisme comprenant un clapet et un film en polymère ;
- la figure 3 représente une seconde alternative du deuxième mode de réalisation du mécanisme de fermeture, ledit mécanisme comprenant un clapet, et des particules de polymère ;
- la figure 4 représente un troisième mode de réalisation du mécanisme de fermeture, ledit mécanisme comprenant, un clapet, et deux polymères sous forme de poudre ou de film ;
- la figure 5 représente un quatrième mode de réalisation du mécanisme de fermeture, ledit mécanisme comprenant un clapet comprenant des plots ;
- la figure 5A représente une vue de dessus du mécanisme de fermeture représenté dans la figure 5 ;
- la figure 6 représente un cinquième mode de réalisation du mécanisme de fermeture, ledit mécanisme comprenant un clapet comprenant un bouton pression ;
- la figure 6A représente une vue de dessus du mécanisme de fermeture représenté dans la figure 6 ;
- la figure 7 représente un sixième mode de réalisation du mécanisme de fermeture, ledit mécanisme comprenant un clapet, ledit clapet comprenant un bouton pression et étant lié à l'enveloppe ;
- la figure 7A représente un agrandissement du mécanisme de fermeture représenté dans la figure 7 ;
- la figure 8 représente un septième mode de réalisation du mécanisme de fermeture comprenant un obturateur ;
- la figure 9 représente un huitième mode de réalisation du mécanisme de fermeture comprenant un clapet sous forme d'une coupelle ; et
- la figure 9A représente une vue de dessus du mécanisme de fermeture représenté dans la figure 9.

De préférence, les mécanismes de fermeture selon les modes de réalisation 1 à 7 et 9 comprennent un clapet 4. Ce clapet est généralement étanche au fluide intestinal. Par « clapet étanche au fluide intestinal », on entend qu'à 37°C, le fluide intestinal ne passe pas à travers le clapet. De préférence, le clapet est en polyéthylène ou en silicone. Le clapet peut être sous forme de disque qui, une fois que le mécanisme de fermeture est fermé, obstrue complètement l'orifice 3. De préférence, le clapet est un disque présentant une épaisseur comprise entre 0,1 mm et 0,4 mm. De préférence, la surface du clapet est supérieure à la surface de l'orifice.

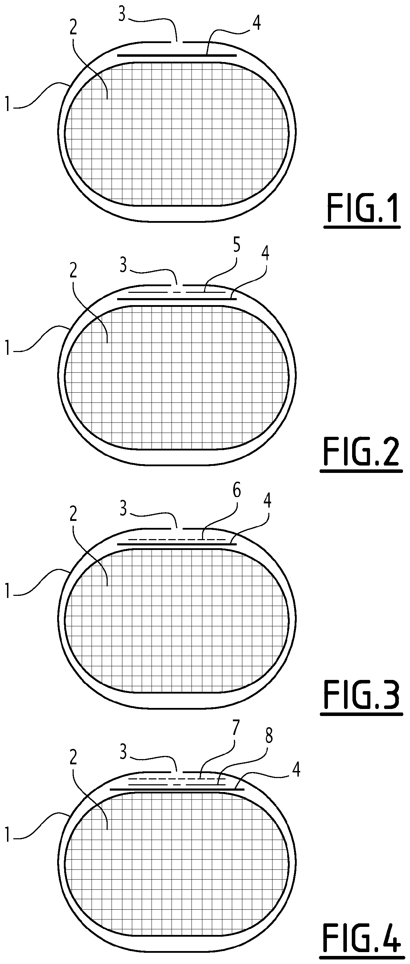

Selon un premier mode de réalisation illustré par la figure 1, le mécanisme de fermeture de l'orifice 3 comprend un clapet 4 étanche au fluide intestinal, le clapet étant intercalé entre l'enveloppe 1 et le matériau interne 2 et disposé sous l'orifice 3.

Selon un deuxième mode de réalisation, le mécanisme de fermeture de l'orifice 3 du dispositif de prélèvement peut comprendre :
- un clapet 4 étanche au fluide intestinal, le clapet étant intercalé entre l'enveloppe 1 et le matériau interne 2 et disposé sous l'orifice 3 ;
- un film 5 ou des particules 6 de diamètre moyen compris entre 0,10mm et 2mm, ledit film 5 ou lesdites particules 6 comprenant un polymère susceptible de se dissoudre à 37°C dans le fluide intestinal, ledit film 5 ou lesdites particules 6 étant disposé(es) entre le clapet 4 et l'enveloppe 1,
où le film 5 ou les particules 6 et l'enveloppe 1 forment ensemble des canaux par lesquels le fluide intestinal est susceptible de circuler et d'atteindre le matériau interne 2.

Plus particulièrement, selon une première alternative du deuxième mode de réalisation représenté sur la figure 2, le mécanisme de fermeture de l'orifice 3 du dispositif comprend :
- un clapet 4 étanche au fluide intestinal, le clapet étant intercalé entre l'enveloppe 1 et le matériau interne 2 et disposé sous l'orifice 3 ;
- un film 5 comprenant un polymère susceptible de se dissoudre à 37°C dans le fluide intestinal, le film étant disposé entre le clapet 4 et l'enveloppe 1,
où le film 5 et l'enveloppe 1 forment ensemble des canaux par lesquels le fluide intestinal est susceptible de circuler et d'atteindre le matériau interne.

Le polymère du film 5 est susceptible de se dissoudre dans le fluide intestinal à 37°C en une durée inférieure à 5 minutes.

De préférence, le polymère du film 5 est biocompatible. De préférence, le polymère du film 5 susceptible de se dissoudre dans le fluide intestinal à 37°C est un polymère porteur de fonctions hydroxyle, acide carboxylique ou carboxylate, de préférence le polymère du film 5 est en poly(alcool vinylique) (PVA). De préférence, le film 5 présente une structure gaufrée.

Selon une seconde alternative du deuxième mode de réalisation représenté sur la figure 3, le mécanisme de fermeture de l'orifice du dispositif de prélèvement comprend :
- un clapet 4 étanche au fluide intestinal, le clapet étant intercalé entre l'enveloppe 1 et le matériau interne 2 et disposé sous l'orifice ;
- des particules 6 de diamètre moyen compris entre 0,10 mm et 2 mm disposées entre le clapet 4 et l'enveloppe 1, et comprenant un polymère susceptible de se dissoudre à 37°C dans le fluide intestinal ;
où les particules 6 et l'enveloppe 1 forment ensemble des canaux par lesquels le fluide intestinal est susceptible de circuler et d'atteindre le matériau interne.

Le diamètre moyen des particules 6 peut être déterminé par microscopie électronique à balayage. De préférence, le polymère compris dans les particules 6 est susceptible de se dissoudre dans le fluide intestinal en une durée inférieure à 5 minutes. De préférence, le polymère compris dans les particules est biocompatible. De préférence, ce polymère des particules 6 est un poly(éthylène glycol) (PEG), typiquement un poly(éthylène glycol) de masse moléculaire inférieure à 20 000 g/mol, de préférence de masse moléculaire égale à 8 000 g/mol. Des PEG de masses moléculaires supérieures mettent plus de temps pour se dissoudre dans le fluide intestinal et sont moins adaptés à la présente invention.

Selon un troisième mode de réalisation représenté sur la figure 4, le mécanisme de fermeture de l'orifice 3 comprend :
- un clapet 4 étanche au fluide intestinal, le clapet étant intercalé entre l'enveloppe 1 et le matériau interne 2 et disposé sous l'orifice 3 ;
- un film ou des particules 7 disposé(es) entre le clapet 4 et l'enveloppe 1 et comprenant un premier polymère porteur de fonctions hydroxyle, acide carboxylique et/ou carboxylate ; et
- un film ou des particules 8 disposé(es) entre le film ou les particules 7 et le clapet 4 et comprenant un deuxième polymère porteur de fonctions hydroxyle, acide carboxylique et/ou carboxylate ;
- où le film ou les particules 7 comprenant le premier polymère, le film ou les particules 8 comprenant le deuxième polymère et l'enveloppe 1 forment ensemble des canaux par lesquels le fluide intestinal est susceptible de circuler et d'atteindre le matériau interne 2,
sous réserve que :
- lorsque le premier polymère est porteur de fonctions hydroxyle, le deuxième polymère est porteur de fonctions acide carboxylique et/ou carboxylate,
- lorsque le premier polymère est porteur de fonctions acide carboxylique et/ou carboxylate, le deuxième polymère est porteur de fonctions hydroxyle,
- les premier et deuxième polymères sont susceptibles de réagir ensemble pour former un polyester lors de la circulation du liquide intestinal dans les canaux.

Indifféremment, les premier et deuxième polymères peuvent être sous forme de film ou de particules 7, 8. Le diamètre moyen des particules peut être compris entre 0,10mm et 2mm, et être mesuré par microscopie électronique à balayage. L'épaisseur du film des polymères 7, 8 peut être comprise entre 0,1 mm et 0,5 mm.

Dans ce mode de réalisation, les premier et deuxième polymères sont susceptibles de former un polyester qui obstrue les canaux par lesquels le liquide intestinal circule, et qui obstrue également l'orifice. Le polyester ainsi formé ne se dissout pas dans le fluide intestinal à 37°C, de préférence sa limite de solubilité à 37°C dans le fluide intestinal est inférieure ou égale à 1% massique, de préférence 0% massique. De préférence, les premier et deuxième polymères sont biocompatibles et à l'état déshydraté. De préférence, ces polymères porteurs de fonctions hydroxyle, acide carboxylique et/ou carboxylate sont hydrophiles et totalement solubles dans le fluide intestinal, *i.e.* leur limite de solubilité dans le fluide intestinal à 37°C est supérieure à 95% massique.

De préférence, le polymère porteur de fonctions hydroxyle est le poly(alcool vinylique). Avantageusement, le polymère porteur de fonctions acide carboxylique et/ou carboxylate est choisi parmi l'acide polyacrylique ou l'un de ses sels, de préférence c'est le polyacrylate de sodium.

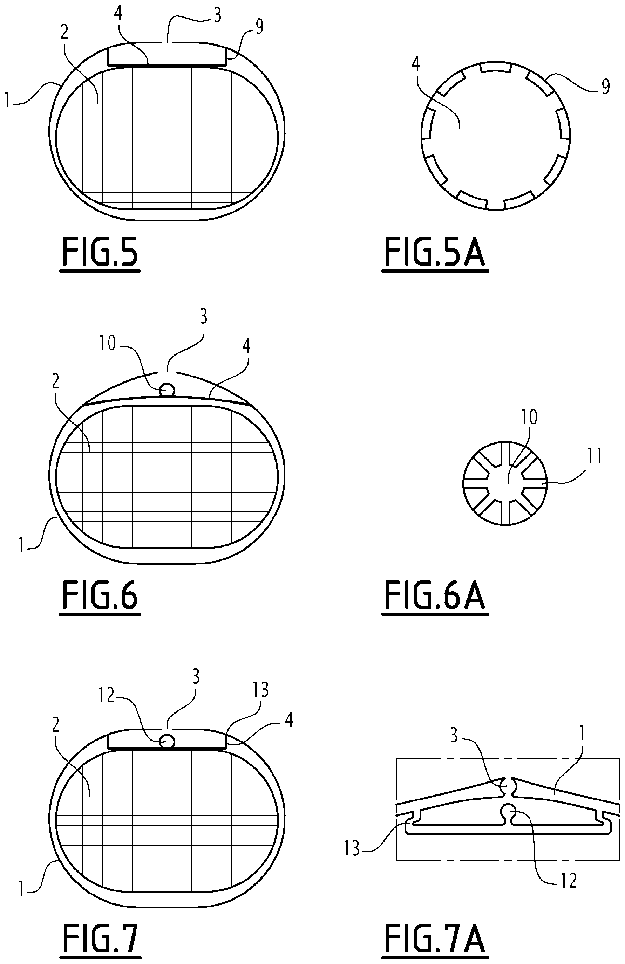

Selon un quatrième mode de réalisation représenté sur la figure 5, le clapet 4 est moulé, possède une forme circulaire et comporte sur sa circonférence des structures d'intercalation 9 pouvant également être moulées. Ces structures d'intercalation représentent des sortes de plots et forment une seule et unique pièce avec le clapet 4. De préférence, le clapet 4 (y compris ses structures d'intercalation 9) comprend le même polymère que celui de l'enveloppe 1, ce qui permet une bonne adhésion entre le clapet 4 et l'enveloppe 1 lorsque le mécanisme de fermeture se ferme. Par exemple, le polymère du clapet 4 et de l'enveloppe 1 est une silicone. Typiquement, le clapet est moulé en silicone de dureté de 30 shore T ce qui lui confère à la fois une souplesse et une rigidité contrôlée ainsi qu'une très bonne adhésion à une enveloppe en silicone.

Selon un cinquième mode de réalisation représenté sur les figures 6 et 6A, le clapet 4 comporte en son centre une excroissance en forme d'un bouton pression 10, pouvant être en polymère. La partie supérieure du bouton pression est structurée sous forme de canaux radians 11.

Selon un sixième mode de réalisation représenté sur la figure 7, le clapet 4 comporte en son centre un bouton pression 12 présentant en périphérie des liaisons ponctuelles 13 avec l'enveloppe 1. Le clapet 4 (y compris le bouton pression 12 et les liaisons ponctuelles 13) comprend le même polymère que celui de l'enveloppe 1, ce qui permet une bonne adhésion entre le clapet 4 et l'enveloppe 1 lorsque le mécanisme de fermeture se ferme. Par exemple, le polymère du clapet 4 et de l'enveloppe 1 est une silicone. L'ensemble clapet 4 et liaisons 13 peut être moulé de façon monobloc avec l'enveloppe 1 de telle sorte qu'il ne peut se déplacer latéralement. Les liaisons 13 assurent que le bouton pression 12 est constamment centré en face de l'orifice 3. La pièce peut être par exemple moulée en silicone de dureté de 30 shore T ce qui lui confère à la fois une souplesse et une rigidité contrôlée ainsi qu'une très bonne adhésion à une enveloppe en silicone.

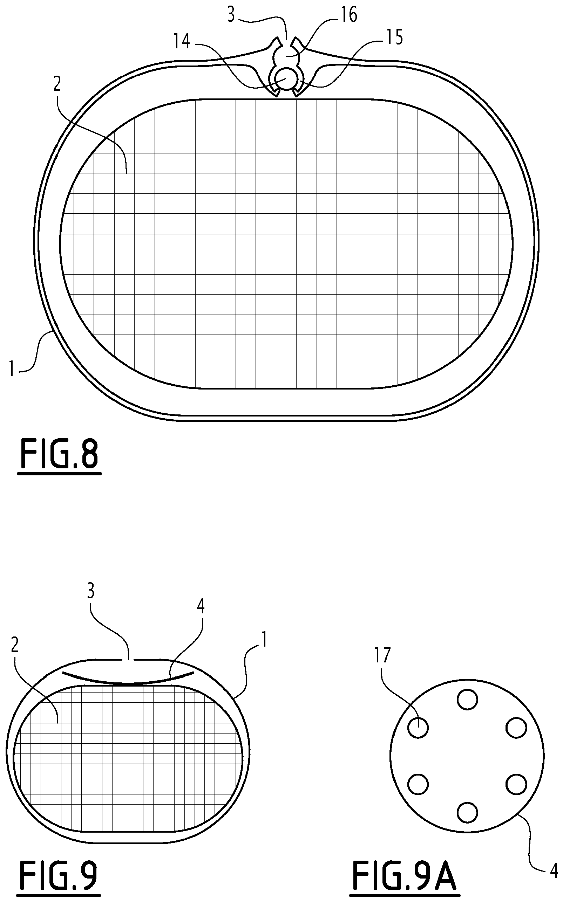

Selon un septième mode de réalisation représenté sur la figure 8, l'orifice 3 est tel qu'il peut recevoir une petite bille 14, par exemple en verre ou en polystyrène, ayant typiquement un diamètre d'environ 1 mm, l'ensemble jouant le rôle d'un obturateur. Cet obturateur est constitué de deux chambres inférieure 15 et supérieure 16 pouvant recevoir la bille 14, la chambre inférieure 15 étant plus large que la chambre supérieure 16. Lorsque la bille se trouve dans la chambre inférieure 15, l'orifice est ouvert. Lorsque la bille se trouve dans la chambre supérieure 16, l'orifice est fermé.

Selon un huitième mode de réalisation représenté sur la figure 9, le clapet 4 a la forme d'une coupelle concave, dont le pourtour est ajouré par des perforations 17 permettant l'admission du fluide intestinal au travers l'orifice 3.

Les différents systèmes de fermeture 5 à 9 décrits ci-dessus peuvent tous comprendre un film 5, 7, 8 ou des particules 6, 7, 8 comprenant un polymère permettant de former avec l'enveloppe des canaux par lesquels le fluide intestinal est susceptible de circuler et d'atteindre le matériau interne, facilitant l'admission du fluide intestinal à l'intérieur du dispositif. Ce film 5, 7, 8 et ces particules 6, 7, 8 en polymère sont notamment tels que définis ci-dessus dans les modes de réalisation 2, 3 et 4.

Comme expliqué ci-après, le matériau interne, en gonflant, participe à la fermeture du système de fermeture en exerçant une force sur un de ses composants. De préférence, le système de fermeture de l'orifice du dispositif ne comprend pas de matériau interne.

La présente invention concerne également un procédé (P1) de prélèvement d'un échantillon de fluide intestinal comprenant :
i) l'ingestion d'au moins un dispositif de prélèvement d'un échantillon de fluide intestinal tel que défini ci-dessus ;
ii) le transit du au moins un dispositif jusqu'à l'intestin, ce par quoi le matériau gastro-résistant se dissout, ce qui libère l'(les) orifice(s) ;
iii) l'admission du fluide intestinal au sein de l'enveloppe à travers l'(les) orifice(s), ce par quoi le matériau interne gonfle par absorption du fluide intestinal à un volume supérieur ou égal à 1,1 × Vi ;
iv) la fermeture du mécanisme de fermeture de l'orifice ;
v) la récupération du(des) dispositif(s) dans les fèces.

Le procédé (P1) peut être appliqué à l'humain ou l'animal.

Lors de l'étape ii) du procédé (P1), le dispositif passe par l'estomac et se retrouve en présence de fluide gastrique. Le dispositif est stable en présence de fluide gastrique grâce notamment au fait qu'un matériau gastro-résistant et susceptible de se dissoudre dans le fluide intestinal à 37°C est disposé au-dessus de chaque orifice, et que l'enveloppe est étanche au fluide gastrique. Cela signifie que le dispositif ne se déforme pas et ne se désagrège pas au contact du fluide gastrique. A la fin de l'étape ii) du procédé, le dispositif se retrouve dans les intestins et est donc au contact du fluide intestinal, ce qui provoque la dissolution du matériau gastro-résistant, et rend l'orifice accessible au fluide intestinal, rendant possible l'étape iii). Lors de l'étape iii), l'orifice est au moins partiellement ouvert, de préférence il est totalement ouvert. L'orifice est totalement ouvert lorsque tout le matériau gastro-résistant qui obturait l'orifice a été dissout au contact du fluide intestinal.

Lors de l'étape iii), le fluide intestinal est admis au sein de l'enveloppe à travers l'orifice, et plus particulièrement dans la structure alvéolaire ouverte du matériau interne qui gonfle, ce par quoi le fluide intestinal est prélevé.

La structure alvéolaire ouverte du matériau interne permet une libre circulation du fluide intestinal. L'absorption du fluide intestinal au sein du matériau interne conduit à son gonflement jusqu'à atteindre un volume supérieur ou égal à 1,1 x Vᵢ, de préférence supérieur ou égal à 2 x Vᵢ, de préférence supérieur ou égal à 6 x Vᵢ.

L'admission de fluide intestinal dans le dispositif de prélèvement lors de l'étape iii) génère une force motrice de gonflement. Cette force de gonflement est typiquement calculée de façon à être adaptée à la contre force exercée par l'enveloppe qui enveloppe le matériau interne du fait de son élasticité. Ceci permet de définir de manière précise en amont du procédé (P1) les paramètres de taille, de forme et de volume du dispositif de prélèvement.

Le procédé (P1), et notamment l'étape iii), repose donc sur un équilibre de forces entre le matériau interne et l'enveloppe. Avant d'être ingéré, le matériau interne au moins partiellement compressé reste compressé même si aucune force ne lui est opposée. Lors de l'étape iii), le matériau interne absorbe le fluide intestinal et gonfle. Lors du gonflement, les alvéoles de la structure alvéolaire connectées entre elles se déploient, constituant ainsi un réservoir de fluide intestinal, et *a fortiori* de microbiote. Le fluide intestinal absorbé par le matériau interne exerce une force de gonflement centrifuge qui est en permanence compensée par la contre force centripète de l'enveloppe qui le contraint. L'ensemble, opposant d'une part une réserve de pression de gonflement du matériau interne et d'autre part la tension de contrainte de l'enveloppe, fournit le moteur qui est nécessaire au prélèvement de fluide intestinal.

De manière avantageuse, le dispositif est capable de conserver durant la durée du transit intestinal le fluide intestinal prélevé et contenant le microbiote qui sera analysé ultérieurement. De façon avantageuse, le dispositif permet de conserver le microbiote vivant.

Le matériau gastro-résistant se présente généralement sous forme d'une couche disposé au-dessus de chaque orifice. De manière avantageuse, le moment et le lieu de prélèvement du fluide intestinal sont prédéterminés par le pH, l'épaisseur et/ou la quantité de la couche de matériau gastro-résistant disposée au-dessus de la surface externe de l'orifice, déterminant ainsi la durée de sa dissolution. Ainsi une faible épaisseur de la couche de matériau gastro-résistant, ou une faible quantité de ce matériau permet une dissolution rapide et donc un prélèvement rapide. A l'inverse une grande épaisseur de la couche de matériau gastro-résistant, ou une quantité importante, retarde sa dissolution complète et favorise un prélèvement plus lent. La combinaison de l'épaisseur et/ou la quantité de la couche de matériau gastro-résistant avec la solubilité de ce dernier en fonction du pH permet de prédéterminer le lieu exact du prélèvement du fluide intestinal.

Généralement, la réalisation de l'étape iii) du procédé (P1) déclenche l'étape iv). En d'autres termes, l'admission du fluide intestinal au sein du dispositif provoque le gonflement du matériau interne qui induit la fermeture du mécanisme de fermeture de l'(les)orifice(s). De manière avantageuse, l'étape iv) du procédé (P1) est déclenchée lorsque que le dispositif est au moins partiellement rempli de fluide intestinal. Le mécanisme de fermeture de l'(les) orifice(s) est actionné par gonflement du matériau interne au contact du fluide intestinal. Ainsi, la fermeture du mécanisme de fermeture est réalisée grâce à l'équilibre de la force de gonflement et de la contre force de l'enveloppe.

De préférence, la durée totale des étapes iii) et iv) est inférieure à 5 min.

La fermeture du mécanisme de fermeture permet que l'échantillon de fluide intestinal prélevé ne soit pas contaminé ultérieurement au cours du transit intestinal.

Dans le premier mode de réalisation du mécanisme de fermeture de l'orifice 3, lors de l'étape iii), le matériau interne 2 gonfle, exerçant alors une pression sur le clapet 4 qui se déforme pour s'adapter à la forme de l'enveloppe 1. Plus le volume de fluide intestinal admis dans le dispositif augmente, plus la pression exercée par le matériau interne 2 sur le clapet 4 augmente, ce qui renforce le contact entre le clapet 4 et l'enveloppe 1, permettant d'obstruer efficacement l'orifice 3. Dans le cas particulier où l'enveloppe 1 et le clapet 4 sont constitués de silicone, la cohésion entre le clapet 4 et l'enveloppe 1 est accentuée par la consistance gommeuse et par les interactions hydrophobes entre ceux-ci.

Dans la première alternative du deuxième mode de réalisation du mécanisme de fermeture de l'orifice 3, le film 5 et l'enveloppe 1 forment ensemble des canaux par lesquels le fluide intestinal est susceptible de circuler et d'atteindre le matériau interne, ce qui permet l'admission du fluide intestinal à l'intérieur du dispositif. Lorsque le fluide intestinal circule dans les canaux, le film en polymère 5 se dissout, les canaux disparaissent et le clapet 4 se colle à l'orifice 3, ce qui conduit à la fermeture de l'orifice 3.

Dans la seconde alternative du deuxième mode de réalisation du mécanisme de fermeture de l'orifice 3, les particules 6 et l'enveloppe 1 forment ensemble des canaux par lesquels le fluide intestinal est susceptible de circuler et d'atteindre le matériau interne, ce qui permet l'admission du fluide intestinal à l'intérieur du dispositif. Lorsque le fluide intestinal circule dans les canaux, les particules 6 en polymère se dissolvent, les canaux disparaissent et le clapet 4 se colle à l'orifice 3, ce qui conduit à la fermeture de l'orifice 3.

Dans le troisième mode de réalisation du mécanisme de fermeture de l'orifice 3, lors de l'étape iii) du procédé (P1), les polymères du film et/ou des particules 7 et 8 se solubilisent au contact du liquide intestinal, et réagissent entre eux par une réaction d'estérification. Le polyester ainsi formé durcit et permet d'obstruer efficacement l'orifice 3. La durée de l'estérification est suffisamment longue pour que le prélèvement de fluide intestinal se produise.

Dans le quatrième mode de réalisation du mécanisme de fermeture de l'orifice 3, les plots 9 du clapet 4 maintiennent un espace entre l'enveloppe 1 et le matériau interne 2 ce qui permet l'entrée du fluide intestinal, ce par quoi le matériau interne 1 gonfle. Grâce à la pression dégagée grâce au gonflement du matériau interne, le clapet 4 s'incurve pour s'adapter parfaitement à la forme de l'enveloppe 1, de telle sorte que l'orifice 3 se trouve parfaitement obstrué.

Dans le cinquième mode de réalisation du mécanisme de fermeture de l'orifice 3, les canaux radians 11 permettent l'entrée du fluide intestinal par l'orifice 3, tout en maintenant l'enveloppe 1 écartée du matériau interne 2. La pression dégagée grâce au gonflement du matériau interne permet au bouton pression 10 de s'introduire dans l'orifice 3 et de ce fait l'obstrue de façon non réversible, rendant l'entièrement du dispositif étanche au fluide intestinal.

Dans le sixième mode de réalisation du mécanisme de fermeture de l'orifice 3, la pression dégagée grâce au gonflement du matériau interne permet d'incurver le clapet 4 de telle sorte que le bouton pression 12 se trouvant face à l'orifice 3 s'engage dans celui-ci et l'obstrue.

Dans le septième mode de réalisation du mécanisme de fermeture de l'orifice 3, lorsque la pression dégagée grâce au gonflement du matériau interne augmente, la bille 14 remonte dans la chambre 16 ce qui obstrue l'orifice 3. Lorsque la pression augmente encore, la bille 14 ne peut pas passer par l'orifice 3 et verrouille ainsi l'orifice de façon irréversible.

Dans le huitième mode de réalisation du mécanisme de fermeture de l'orifice 3, l'action combinée du gonflement du matériau interne 2 et de l'étirement de l'enveloppe 1, tend à écarter les bords du clapet 4, donc de l'aplatir et pousse progressivement le clapet en direction de l'enveloppe 1 ce qui inverse sa concavité. En se plaquant sur l'enveloppe 1, le clapet 4 occulte hermétiquement l'orifice 3.

De manière avantageuse, le suivi du dispositif de prélèvement pendant les étapes i) à iv) du procédé (P1) est possible grâce à la présence d'un fil métallique ferromagnétique compris dans l'épaisseur de l'enveloppe. Ce suivi peut être réalisé par radiographie ou échographie.

De manière avantageuse, l'étape v) du procédé (P1) est facilitée par la présence du fil métallique ferromagnétique compris dans l'épaisseur de l'enveloppe, la récupération étant alors réalisée grâce à un aimant ou tout autre dispositif magnétique.

A l'issue de l'étape v), le liquide intestinal prélevé dans le dispositif peut être caractérisé et le microbiote analysé par toute technique connue de l'homme du métier. De préférence, les alvéoles de la structure alvéolaire ouverte du dispositif récupéré ont un diamètre moyen interne tel que mesuré par microscopie électronique à balayage de 10µm à 3mm. De manière avantageuse, la plus grande dimension (diamètre ou hauteur) du dispositif dépend du sujet sur lequel le dispositif est utilisé. Ainsi, à l'issue de l'étape v), si le dispositif est destiné à être utilisé chez un petit animal de type rat, la plus grande dimension du dispositif est inférieure à 2mm. Quand le dispositif est utilisé chez l'homme, la plus grande dimension est généralement inférieure à 10mm, de préférence inférieure à 8mm.

La présente invention concerne également l'utilisation du dispositif pour le prélèvement de fluide intestinal. Le dispositif de prélèvement peut avantageusement être utilisé pour prélever le fluide intestinal à un moment et un lieu prédéterminé.

L'invention concerne également un procédé (P2) de préparation du dispositif comprenant les étapes suivantes :
a) Fournir un matériau en polymère ayant un volume V₀ supérieur à Vi,
b) Compresser au moins partiellement ledit matériau en polymère jusqu'à atteindre un volume Vᵢ,
c) Envelopper ledit matériau en polymère au moins partiellement comprimé obtenu à l'étape b) dans une enveloppe,
d) Appliquer un matériau gastro-résistant et susceptible de se dissoudre dans le fluide intestinal à 37°C à la surface externe de chaque orifice,
α) Intégrer un mécanisme de fermeture de l'(des) orifice(s),
l'étape a) pouvant être réalisée avant, pendant ou après l'étape c).

Le matériau fourni à l'étape a) est un matériau correspondant au matériau interne défini ci-dessus, sauf qu'il a un volume V₀ supérieur à Vᵢ, ce qui signifie donc qu'il est moins compressé que le matériau interne du dispositif, de préférence il n'est pas compressé. De préférence, V₀ est supérieur ou égal à 2 × Vᵢ, de préférence supérieur ou égal à 4 × Vᵢ. Typiquement, V₀ est compris entre 4 × Vᵢ et 6 × Vᵢ.

Le matériau en polymère ayant un volume V₀ est généralement un matériau commercial. On peut citer par exemple des matériaux en poly(alcool vinylique) sous forme de mousse compris dans des mèches chirurgicales utiles pour éponger le sang lors d'opération chirurgicales.

Typiquement, l'étape b) est réalisée au moyen d'une pince ou d'une presse hydraulique selon la dimension maximale du dispositif. La pression appliquée est de l'ordre de 5 kg/cm². Généralement, le matériau en polymère au moins partiellement compressé obtenu à la fin de l'étape b) reste au volume Vᵢ à la fin de l'étape b), et ce même lorsque la force de compression n'est plus appliquée. Il reste en forme.

Typiquement, l'étape c) est réalisée par tout moyen connu de l'homme du métier.

L'étape a) du procédé (P2) peut être réalisée avant, pendant ou après l'étape c). Ainsi, le ou les orifices peuvent déjà être présents sur l'enveloppe lors de l'enveloppage du matériau en polymère ; ou ils peuvent être formés avant ou après avoir disposé l'enveloppe.

Typiquement, l'étape d) du procédé (P2) est réalisée par tout moyen connu de l'homme du métier.

### EXEMPLE : Préparation d'un dispositif selon l'invention

On a travaillé dans un environnement propre, stérile et exempt de poussières. Le dispositif a été préparé à partir d'un matériau alvéolaire en éponge de poly(alcool vinylique) (PVA) tel que ceux parfois utilisés par les chirurgiens pour éponger un champ opératoire. Un échantillon de ce matériau ayant une épaisseur de 10mm a été placé sur une surface solide. A l'aide d'un emporte-pièce coupant de 10mm de diamètre, on a découpé et détaché un cylindre au format 10x10mm. Ce cylindre a ensuite été compressé à l'aide d'une pince pour obtenir un disque de format 10x2mm. Avec le même emporte-pièce on a découpé un clapet dans un film de polyéthylène de 0,2mm d'épaisseur que l'on a superposé au disque comprimé. L'ensemble a été placé dans une enveloppe faite d'un doigt de gant chirurgical en copolymère butadiène-acrylonitrile. Le doigt de gant a été noué pour former une enveloppe hermétique tout en repérant à l'intérieur la position du clapet. A l'aide d'une pince fine pointue, on a effectué un orifice de 1 mm de diamètre dans l'enveloppe en copolymère butadiène-acrylonitrile au-dessus du centre du clapet sans endommager ce dernier. Ce dispositif est caractérisé par des dimensions adaptées à une expérimentation animale sur le cochon.
Les volumes suivants ont été mesurés :

| | |
|---|---|
| Volume du matériau | V₀ = 0,785ml |
| Volume compressé | Vᵢ = 0,157ml |
| Volume du matériau après gonflement | V = 0,470ml |
| Volume de fluide intestinal collecté | V = 0,310ml |

L'enveloppe en nitrile présentait une dureté de 50 Shore et un module de Young de 1,5MPa. Une fois compressé, le diamètre moyen des pores du matériau interne en PVA était compris entre 7 et 12µm. Après prélèvement de fluide intestinal, le diamètre moyen des pores du matériau interne était compris entre 80 et 200µm. L'élongation à l'état humide était de 250%. La porosité se situait entre 89 et 91%. A l'état sec, le volume des pores était de 0,56cm³ g⁻¹.

## Revendications

1. Dispositif de prélèvement d'un échantillon de fluide intestinal comprenant :
- une enveloppe (1) en polymère élastique et étanche au fluide digestif, ladite enveloppe comprenant au moins un orifice (3) permettant l'admission du fluide intestinal au sein du dispositif;
- un matériau gastro-résistant et susceptible de se dissoudre dans le fluide intestinal à 37°C étant disposé au-dessus de chaque orifice ; et
- au moins un mécanisme de fermeture du ou des orifices, **caractérisé en ce que**: le dispositif comprend en outre un matériau interne en polymère présentant une structure alvéolaire ouverte au moins partiellement compressée, ayant un volume Vᵢ et apte à gonfler à un volume supérieure ou égal à 1,1 x Vᵢ par absorption de fluide intestinal, l'enveloppe enveloppant le matériau interne (2), ledit au moins un orifice (3) permettant l'admission du fluide intestinal au sein du matériau interne, de sorte que le gonflement du matériau interne provoque une déformation élastique de l'enveloppe, et **en ce que**: le mécanisme de fermeture du ou des orifices est configuré de telle sorte que le gonflement du matériau interne provoqué par l'admission du fluide intestinal au sein du dispositif induit la fermeture du mécanisme de fermeture du ou des orifices.

2. Dispositif selon la revendication 1 dans lequel le polymère de l'enveloppe (1) est choisi parmi le latex, les polyvinyles, les polymères nitrile, le silicone, et un mélange de ceux-ci.

3. Dispositif selon l'une quelconque des revendications précédentes dans lequel le matériau interne (2) est susceptible de gonfler à un volume supérieur ou égal à 2 x Vᵢ, de préférence supérieur ou égale à 6 x Vᵢ par absorption de fluide intestinal.

4. Dispositif selon l'une quelconque des revendications précédentes dans lequel le polymère du matériau interne (2) est choisi parmi les poly(alcool vinylique) au moins partiellement réticulés, les acides poly(méth)acryliques au moins partiellement réticulés et leurs sels, les polysaccharides au moins partiellement réticulés, ou un mélange de ceux-ci.

5. Dispositif selon l'une quelconque des revendications précédentes dans lequel le mécanisme de fermeture de l'orifice (3) comprend un clapet (4) étanche au fluide intestinal, le clapet étant intercalé entre l'enveloppe (1) et le matériau interne (2) et disposé sous l'orifice (3).

6. Dispositif selon la revendication 5 dans lequel le mécanisme de fermeture de l'orifice (3) comprend un film (5) ou des particules (6) de diamètre moyen compris entre 0,10mm et 2mm, ledit film (5) ou lesdites particules (6) comprenant un polymère susceptible de se dissoudre à 37°C dans le fluide intestinal, ledit film (5) ou lesdites particules (6) étant disposé(es) entre le clapet (4) et l'enveloppe (1), où le film (5) ou les particules (6) et l'enveloppe (1) forment ensemble des canaux par lesquels le fluide intestinal est susceptible de circuler et d'atteindre le matériau interne (2).

7. Dispositif selon la revendication 5 dans lequel le mécanisme de fermeture de l'orifice (3) comprend :
- un film ou des particules (7) disposé(es) entre le clapet (4) et l'enveloppe (1) et comprenant un premier polymère porteur de fonctions hydroxyle, acide carboxylique et/ou carboxylate ; et
- un film ou des particules (8) disposé(es) entre le film ou les particules (7) et le clapet (4) et comprenant un deuxième polymère porteur de fonctions hydroxyle, acide carboxylique et/ou carboxylate ;
- où le film ou les particules (7) comprenant le premier polymère, le film ou les particules (8) comprenant le deuxième polymère et l'enveloppe (1) forment ensemble des canaux par lesquels le fluide intestinal est susceptible de circuler et d'atteindre le matériau interne (2),
sous réserve que :
- lorsque le premier polymère est porteur de fonctions hydroxyle, le deuxième polymère est porteur de fonctions acide carboxylique et/ou carboxylate,
- lorsque le premier polymère est porteur de fonctions acide carboxylique et/ou carboxylate, le deuxième polymère est porteur de fonctions hydroxyle,
- les premier et deuxième polymères sont susceptibles de réagir ensemble pour former un polyester lors de la circulation du liquide intestinal dans les canaux.

8. Dispositif selon l'une quelconque des revendications précédentes susceptible de prélever un volume de fluide intestinal compris entre 0,01mL et 1mL, de préférence un volume de fluide intestinal égal à 0,3mL.

9. Procédé (P1) de prélèvement d'un échantillon de fluide intestinal comprenant :
i) l'ingestion d'au moins un dispositif selon l'une quelconque des revendications précédentes ;
ii) le transit du au moins un dispositif jusqu'à l'intestin, ce par quoi le matériau gastro-résistant se dissout, ce qui libère l'(es) orifice(s)
iii) l'admission du fluide intestinal au sein de l'enveloppe à travers l'(les) orifice(s) (3), ce par quoi le matériau interne (2) gonfle par absorption du fluide intestinal à un volume supérieur ou égal à 1,1 × Vi ;
iv) la fermeture du mécanisme de fermeture de l'orifice (3) ;
v) la récupération du(des) dispositif(s) dans les fèces.

10. Utilisation du dispositif selon l'une quelconque des revendications 1 à 8 pour le prélèvement de fluide intestinal.

## Patentansprüche

1. Vorrichtung zum Sammeln einer Darmflüssigkeitsprobe, Folgendes umfassend:
- eine Hülle (1) aus elastischem und gegenüber der Verdauungsflüssigkeit dichtem Polymer, wobei die Hülle mindestens eine Öffnung (3) umfasst, welche den Einlass der Darmflüssigkeit innerhalb der Vorrichtung ermöglicht;
- ein gastroresistentes Material, und welches imstande ist, sich in der Darmflüssigkeit bei 37°C aufzulösen, welches oberhalb jeder Öffnung angeordnet ist; und
- mindestens einen Schließmechanismus der Öffnung(en), **dadurch gekennzeichnet, dass**: die Vorrichtung weiter ein internes Material aus Polymer umfasst, welches eine offene wabenförmige mindestens teilweise zusammengedrückte Struktur vorweist, welche ein Volumen Vᵢ aufweist, und in der Lage ist, durch Absorption der Darmflüssigkeit auf ein Volumen größer oder gleich 1,1 x Vᵢ aufzuquellen, wobei die Hülle das interne Material (2) einhüllt, wobei die mindestens eine Öffnung (3) den Einlass der Darmflüssigkeit innerhalb des internen Materials ermöglicht,
derart, dass Aufquellen des internen Materials für eine elastische Verformung der Hülle sorgt, und dadurch, dass: der Schließmechanismus der Öffnung(en) derart konfiguriert ist, dass das Aufquellen des internen Materials, das durch den Einlass der Darmflüssigkeit innerhalb der Vorrichtung verursacht wird, das Schließen des Schließmechanismus der Öffnung(en) veranlasst.

2. Vorrichtung nach Anspruch 1, wobei das Polymer der Hülle (1) ausgewählt ist aus Latex, Polyvinylen, Nitrilpolymeren, Silikon, und einem Gemisch derselben.

3. Vorrichtung nach einem der vorstehenden Ansprüche, wobei das interne Material (2) imstande ist, durch Absorption der Darmflüssigkeit auf ein Volumen größer oder gleich 2 x Vᵢ, vorzugsweise größer oder gleich 6 x Vᵢ aufzuquellen.

4. Vorrichtung nach einem der vorstehenden Ansprüche, wobei das Polymer des internen Materials (2) aus mindestens teilweise vernetztem Poly(Vinylakolohl), mindestens teilweise vernetzten Poly(methyl)acrylsäuren und deren Salzen, den mindestens teilweise vernetzten Polysacchariden, oder einem Gemisch derselben ausgewählt ist.

5. Vorrichtung nach einem der vorstehenden Ansprüche, wobei der Schließmechanismus der Öffnung (3) eine gegenüber Darmflüssigkeit dichte Klappe (4) umfasst, wobei die Klappe zwischen der Hülle (1) und dem internen Material (2) eingesetzt, und unter der Öffnung (3) angeordnet ist.

6. Vorrichtung nach Anspruch 5, wobei der Schließmechanismus der Öffnung (3) einen Film (5) oder Teilchen (6) mit einem mittleren Durchmesser umfasst, der zwischen 0,10mm und 2mm enthalten ist, wobei der Film (5) oder die Teilchen (6) ein Polymer umfassen, das imstande ist, sich bei 37°C in der Darmflüssigkeit aufzulösen, wobei der Film (5) oder die Teilchen (6), der (die) zwischen der Klappe (4) und der Hülle (1) angeordnet ist (sind), wo der Film (5) oder die Teilchen (6) und die Hülle (1) gemeinsam Kanäle bilden, durch welche die Darmflüssigkeit imstande ist, zu zirkulieren, und das interne Material (2) zu erreichen.

7. Vorrichtung nach Anspruch 5, wobei der Schließmechanismus der Öffnung (3) Folgendes umfasst:
- einen Film oder Teilchen (7), der (die) zwischen der Klappe (4) und der Hülle (1) angeordnet ist (sind) und ein erstes Polymer umfassend, welches Träger von Hydroxyl-, Carboxyl- und/ oder Carboxylatsäurefunktionen ist; und
- einen Film oder Teilchen (8), der (die) zwischen dem Film oder den Teilchen (7) und der Klappe (4) angeordnet ist (sind) und ein zweites Polymer umfassend, welches Träger von Hydroxyl-, Carboxyl- und/ oder Carboxylatsäurefunktionen ist;
- wobei der Film oder die Teilchen (7), die das erste Polymer umfassen, der Film oder die Teilchen (8), die das zweite Polymer umfassen, und die Hülle (1) gemeinsam Kanäle bilden, durch die die Darmflüssigkeit imstande ist, zu zirkulieren und das interne Material (2) zu erreichen,
unter dem Vorbehalt, dass:
- wenn das erste Polymer Träger von Hydroxylfunktionen ist, das zweite Polymer Träger von Carboxyl- und/ oder Carboxylatsäurefunktionen ist,
- wenn das erste Polymer Träger von Carboxyl- und/ oder Carboxylatsäurefunktionen ist, das zweite Polymer Träger von Hydroxylfunktionen ist,
- das erste und zweite Polymer imstande sind, gemeinsam zu reagieren, um bei der Zirkulation der Darmflüssigkeit in den Kanälen ein Polyester zu bilden.

8. Vorrichtung nach einem der vorstehenden Ansprüche, die imstande ist, ein Darmflüssigkeitsvolumen, das zwischen 0,01 mL und 1 mL enthalten ist, vorzugsweise ein Darmflüssigkeitsvolumen gleich 0,3mL zu sammeln.

9. Verfahren (P1) zum Sammeln einer Darmflüssigkeitsprobe, Folgendes umfassend:
i) das Verschlucken mindestens einer Vorrichtung nach einem der vorstehenden Ansprüche,
ii) den Transit der mindestens einen Vorrichtung bis zum Darm, wodurch sich das gastroresistente Material auflöst, wodurch die Öffnung(en) freigegeben wird (werden)
iii) den Einlass der Darmflüssigkeit innerhalb der Hülle durch die Öffnung(en) (3) hindurch, wodurch das interne Material (2) durch Absorption der Darmflüssigkeit auf ein Volumen größer oder gleich 1,1 x Vᵢ aufquillt;
iv) das Schließen des Schließmechanismus der Öffnung (3);
v) das Auffangen der Öffnung(en) im Kot.

10. Verwendung der Vorrichtung nach einem der Ansprüche 1 bis 8 zum Sammeln von Darmflüssigkeit.

## Claims

1. Device for collecting an intestinal fluid sample comprising:
- an envelope (1) made of elastic polymer and impervious to digestive fluid, said envelope comprising at least one opening (3) for admitting the intestinal fluid into the device;
- a gastro-resistant material capable of dissolving in the intestinal fluid at 37°C being arranged above each opening; and
- at least one mechanism for closing the opening(s), **characterised in that**: the device further comprises an inner polymeric material having an open cellular structure which is at least partially compressed, having a volume Vᵢ and capable of swelling to a volume greater than or equal to 1.1 x Vᵢ by absorbing intestinal fluid, the envelope enveloping the inner material (2), said at least one opening (3) allowing the admission of intestinal fluid into the inner material,
such that the swelling of the inner material causes an elastic deformation of the envelope, and **in that**: the mechanism for closing the opening(s) is configured such that the swelling of the inner material caused by the admission of intestinal fluid into the device induces the closure of the mechanism for closing the orifice(s).

2. Device according to claim 1 wherein the polymer of the envelope (1) is chosen from latex, polyvinyls, nitrile polymers, silicon, and a mixture thereof.

3. Device according to any one of the preceding claims wherein the inner material (2) is capable of swelling to a volume greater than or equal to 2 x Vᵢ, preferably greater than or equal to 6 x Vᵢ by absorbing intestinal fluid.

4. Device according to any one of the preceding claims wherein the polymer of the inner material (2) is chosen from at least partially crosslinked poly(vinyl alcohols), at least partially crosslinked poly(meth)acrylic acids and salts thereof, at least partially crosslinked polysaccharides, or a mixture thereof.

5. Device according to any one of the preceding claims wherein the mechanism for closing the opening (3) comprises a flap (4) impervious to intestinal fluid, the flap being inserted between the envelope (1) and the inner material (2) and disposed under the opening (3).

6. Device according to claim 5 wherein the mechanism for closing the opening (3) comprises a film (5) or particles (6) of mean diameter between 0.10mm and 2mm, said film (5) or said particles (6) comprising a polymer capable of dissolving at 37°C in intestinal fluid, said film (5) or said particles (6) being disposed between the flap (4) and the envelope (1), where the film (5) or the particles (6) and the envelope (1) together form channels through which intestinal fluid is capable of flowing and reaching the inner material (2).

7. Device according to claim 5 wherein the mechanism for closing the opening (3) comprises:
- a film or particles (7) disposed between the flap (4) and the envelope (1) and comprising a first polymer carrying hydroxyl, carboxylic acid and/or carboxylate functions;
- a film or particles (8) disposed between the film or the particles (7) and the flap (4) and comprising a second polymer carrying hydroxyl, carboxylic acid and/or carboxylate functions;
- where the film or the particles (7) comprising the first polymer, the film or the particles (8) comprising the second polymer and the envelope (1) together form channels through which intestinal fluid is capable of flowing and reaching the inner material (2),
on the condition that:
- when the first polymer is a carrier of hydroxyl functions, the second polymer is a carrier of carboxylic acid and/or carboxylate functions,
- when the first polymer is a carrier of carboxylic acid and/or carboxylate functions, the second polymer is a carrier of hydroxyl functions,
- the first and second polymers are capable of reacting together to form a polyester when intestinal fluid flows in the channels.

8. Device according to any one of the preceding claims capable of collecting a volume of intestinal fluid between 0.01 mL and 1mL, preferably a volume of intestinal fluid equal to 0.3mL.

9. Method (P1) for collecting an intestinal fluid sample comprising:
i) ingesting at least one device according to any one of the preceding claims;
ii) transit of the at least one device to the intestine, whereby the gastro-resistant material dissolves, which frees the opening(s)
iii) admitting intestinal fluid into the envelope through the opening(s) (3), whereby the inner material (2) swells by absorbing intestinal fluid a volume greater than or equal to 1.1 x Vi;
iv) closing the mechanism for closing the opening (3);
v) recovering the device(s) in faeces.

10. Use of the device according to any one of claims 1 to 8 for collecting intestinal fluid.
